# FASCICULE DE BREVET EUROPEEN

(11) **EP 1 395 941 B1**
(45) Date de publication et mention de la délivrance du brevet: **09.08.2006**
(21) Numéro de dépôt: 02751247.4
(22) Date de dépôt: 14.06.2002
(51) Int. Cl.: G06F 19/00

(54) **Méthode d'identification d'un motif ou d'une combinaison de motifs présentant un état booléen de mutations prédéterminées dans un ensemble de séquences et ses applications**
Verfahren zum Identifizieren von Motiven oder von Motivkombinationen, die einen booleschen Zustand von vorherbestimmten Mutationen in einer Menge von Sequenzen darstellen und seine Anwendungen
Method for identifying motifs or combinations of motifs having a boolean state of predefined mutations in a set of sequences and uses thereof

(30) Priorité: 14.06.2001 FR 0107808
(43) Date de publication de la demande: 10.03.2004
(73) Titulaire: CENTRE NATIONAL DE LA RECHERCHE SCIENTIFIQUE (CNRS), 75794 Paris Cedex 16 (FR)
(72) Inventeur: VANET, Anne, F-75019 Paris (FR); MULLER-TRUTWIN, Michaela, F-75003 Paris (FR); Valère, Thomas, F-75019 Paris (FR)
(74) Mandataire: Breese, Pierre
(86) Numéro de dépôt international: PCT/FR2002/002068
(87) Numéro de publication internationale: WO 2002/103605

(56) Documents cités:
- WO-A-99/61658
- SHAFER R W ET AL: "Human immunodeficiency virus type 1 reverse transcriptase and protease mutation search engine for queries" NATURE MEDICINE, [Online] vol. 6, no. 11, November 2000 (2000-11), pages 1290-1292, XP002202300 Retrieved from the Internet: <URL:http://hivdb.stanford.edu/pdf/natureM ed.pdf> [retrieved on 2002-06-13]
- KANTOR R ET AL: "Human Immunodeficiency Virus Reverse Transcriptase and Protease Sequence Database: an expanded data model integrating natural language text and sequence analysis programs" NUCLEIC ACIDS RESEARCH, [Online] vol. 29, no. 1, 1 January 2001 (2001-01-01), pages 296-299, XP002202301 Retrieved from the Internet: <URL:http://nar.oupjournals.org/cgi/conten t/full/29/1/296> [retrieved on 2002-06-13]
- SHAFER R W ET AL: "Human Immunodeficiency Virus Reverse Transcriptase and Protease Sequence Database" NUCLEIC ACIDS RESEARCH, [Online] vol. 28, no. 1, 1 January 2000 (2000-01-01), pages 346-348, XP002202302 Retrieved from the Internet: <URL:http://nar.oupjournals.org/cgi/conten t/full/28/1/346> [retrieved on 2002-06-13]
- SHAFER R W ET AL: "Human Immunodeficiency Virus Reverse Transcriptase and Protease Sequence Database" NUCLEIC ACIDS RESEARCH, [Online] vol. 27, no. 1, 1 January 1999 (1999-01-01), pages 348-352, XP002202303 Retrieved from the Internet: <URL:http://nar.oupjournals.org/cgi/conten t/full/27/1/348> [retrieved on 2002-06-13]

## Description

L'invention appartient au domaine d'analyse des séquences de nucléotides et/ou d'acides aminés composant les organismes vivants, en particulier l'analyse de mutations particulières que lesdites séquences peuvent présenter.

Elle concerne des méthodes d'identification et de sélection de fragments de séquences d'acides nucléiques ou de protéines constitués par et/ou comprenant des motifs présentant des caractéristiques de mutabilité spécifiques, elle concerne également des compositions pharmaceutiques contenant lesdites fragments pour la préparation de médicaments utiles pour le traitement et/ou la prévention, de pathologies humaines, animales et/ou végétales ou pour la préparation de cibles thérapeutiques utiles pour le criblage de composés thérapeutiques.

On connaît que des mutations induites dans les séquences sauvages d'organismes pathogènes sont par exemple, responsables des mécanismes d'échappement thérapeutique, c'est à dire de la capacité des organismes pathogènes, viraux ou bactériens, à résister à un traitement thérapeutique. Les séquences nucléotidiques et/ou polypeptidiques des souches mutantes desdits organismes présentent en effet des mutations particulières par rapport aux séquences nucléotidiques ou polypeptidiques des souches sauvages.

De telles mutations sont également déterminantes de changements fonctionnels des gènes ou des protéines qui ont pour conséquence l'altération de nombreux processus biologiques, tels que le déclenchement de la réponse immune, l'infectivité des virus, l'apparition de cancers, etc.

On connaît, par exemple, que l'information génétique du virus de l'immunodéficience humaine (VIH), appartenant à la famille des rétrovirus, est supportée par deux molécules d'ARN. Lors de l'infection, l'intégration du génome viral à celui de la cellule hôte ne peut donc se faire directement. La synthèse préalable d'une copie d'ADN à partir de l'ARN génomique du virus est une étape déterminante du cycle infectieux. L'enzyme responsable de cette transcription inverse est une protéine appelée Reverse Transcriptase (RT). La faible fidélité reverse-transcriptionnelle de cette dernière confère au virus une grande variabilité génomique. On estime que chez un individu séropositif non traité, une mutation apparaît par réplication, et donc pour les dix milliards de virus produits par jour, il y a 10 milliards de mutation nouvelles. Cette mutation peut entraîner une résistance à un ou plusieurs antirétroviraux et ainsi générer des "souches" plus virulentes car de plus en plus résistantes.

Face à cette problématique, les praticiens prescrivent des traitements très lourds, tels que la trithérapie à long terme, depuis peu la quadrithérapie et peut être plus à l'avenir, profitant de l'absence de virus résistant qui caractérise en général les patients non encore traités et infectés par une seule forme du virus. Ces traitements provoquent alors une forte diminution de la charge virale, considérée comme la quantité de particules virales circulant dans le sang, le nombre de mutants viraux qui est directement proportionnel à la charge virale, diminue également, réduisant ainsi les risques d'échappement thérapeutique.

Malheureusement, ces traitements extrêmement lourds s'accompagnent de nombreux effets secondaires. Ils nécessitent, en outre, une compliance parfaite qui, lorsqu'elle n'est pas respectée, s'accompagne presque systématiquement de l'émergence de souches résistantes. Ces résistances sélectionnées sous la pression des antirétroviraux sont à l'origine de la plupart des échappements thérapeutiques.

Ainsi, alors que le choix d'une combinaison d'antirétroviraux apparaît comme fondamental, l'association optimisée de ces derniers ne semble pas évidente. Outre les problèmes multiples posés par les résistances que nous venons de décrire, l'incompatibilité de certaines associations médicamenteuses et le nombre toujours croissant de molécules antirétrovirales rendent le travail des praticiens de plus en plus ardu.

A l'heure actuelle les médecins disposent d'une vingtaine de composés thérapeutiques, essentiellement dirigés contre deux protéines virales, la reverse transcriptase et la protéase. Les traitements les plus usuels sont les trithérapies. On en dénombre 252 possibles lorsqu'on ne considère que les associations les plus courantes. Ces calculs sont statistiques et ne prennent pas en compte les différentes incompatibilités médicamenteuses. De plus, l'apparition de nouveaux principes actifs issus de la recherche pharmaceutique aura pour conséquence directe de compliquer encore le problème du choix de la combinaison médicamenteuse.

L'activité d'autres organismes pathogènes est tout aussi préoccupante, le virus de la grippe a été responsable de 20 millions de décès durant le XX^{ème} siècle et le virus Ebola émerge de façon alarmante. Les hépatites A, B, C, D et E constituent de véritables priorités de santé publique, de par leur état booléen et leur gravité potentielle.

Or, dans tous les cas il y a un vide thérapeutique et vaccinal qui s'accroît chaque année à cause de la grande mutabilité des génomes viraux: spécialement des rétrovirus, virus à ARN tels que VIH, grippe, Ebola, hépatite C, etc.

Plusieurs approches ont été proposées pour tenter de résoudre ces problèmes de multirésistance liée à la haute mutabilité de certains organismes pathogènes, ainsi, par exemple, la société Virco Tibotech, a développé une méthode gérée par un logiciel qui permet la comparaison d'un génotype donné à toute une banque de séquences VIH. Il définit ensuite la liste des résistances possibles aux composés antirétroviraux.

Aussi, certains sites web, tel que celui de la Los-Alamos Library (http://hiv-web.lanl.gov/) fournissent un grand nombre de données concernant les alignements de séquences protéiques de VIH ainsi que les mutations s'y rapportant.

L'article de SHAFER *et al (Nucleic Acids Research,* vol.28(1), p : 346-348, 2000) décrit la mise en place d'une base de données regroupant les différentes variations de séquences de la transcriptase inverse (RT) et de la protéase de virus du HIV obtenus à partir d'échantillons de patients suivant différents traitements anti-rétroviraux. L'article de KANTOR *et al. (Nucleic Acids Research,* vol.29(1), p : 296-299, 2001) décrit l'évolution de cette même base de données et plus spécifiquement l'intégration d'une méthode d'analyse d'une séquence de référence par rapport aux séquences présentes dans ladite base. Cette méthode d'analyse permet potentiellement au praticien de définir le traitement le plus adapté pour un patient infecté par une souche de virus HIV donné, à laquelle correspond à la séquence de référence, au regard des informations de la base de données concernant l'efficacité des différents traitements anti-rétroviraux sur les souches de HIV dont les séquences sont décrites dans ladite base.

De même, plusieurs publications de Ribeiro *et al.,* divulguent des méthodes mettant en oeuvre de calculs d'état booléens d'apparition de mutants résistants en utilisant des calculs mathématiques assez complexes.

Aussi, des méthodes visant à identifier des mutations des motifs constituante des séquences nucléotidiques ou polypeptidiques ont été développées, par exemple, celles qui ont permis, dans les années 80 de classer les gènes des immunoglobulines en classes et sous-classes, comportant des domaines constants et des domaines variables en fonction de la variabilité de motifs des différentes séquences qui les composent.

Cependant ces méthodes ne permettent pas d'identifier des motifs dont la possibilité de mutation est prédéterminée par rapport à l'ensemble de séquences analysée. Dans le cadre de la présente invention, cette possibilité de mutation correspond à un état booléen de ladite mutation.

La méthode de l'invention a pour objet l'identification de plusieurs motifs dont l'état booléen de mutation relative, par rapport à un ensemble de séquences données, est prédéterminée. Cette méthode est basée sur l'identification soit des motifs ou de combinaisons de motifs n'ayant jamais muté simultanément, soit de motifs ou de combinaison de motifs ayant muté simultanément, au moins une fois sur au moins une des séquences de l'ensemble et n'ayant pas muté sur les autres séquences dudit ensemble.

La présente invention constitue un nouvel outil pour permettre de trouver des solutions plus durables lors des traitements thérapeutiques des pathologies impliquant des organismes pathogènes ou des gènes humains, présentant un haut degré de mutabilité.

Au sens de la présente invention on entend par motif un nucléotide susceptible de faire partie d'une séquence d'acide nucléique ou d'un oligonucléotide synthétique, désigné ci-après par son code unicaractère: A, G, C, T ou U, correspondant à la nomenclature de la base respective (adénine A, guanine G, cytosine C, ou thymine T dans l'ADN, ou uracile U dans l'ARN) dont ils sont constitués.

On entend également par motif un acide aminé, quelle que soit sa configuration, susceptible de faire partie d'une protéine ou d'un peptide naturel ou synthétique, désigné par son code unicaractère tels que par exemple, ceux représentés dans le tableau ci-dessus.

**Code des acides aminés**

| **Code** | **aa** |
|---|---|
| A | Alanine |
| C | Cystéine |
| D | Acide Aspartique |
| E | Acide Glutamique |
| F | Phénylalanine |
| G | Glycine |
| H | Histidine |
| I | Isoleucine |
| K | Lysine |
| L | Leucine |
| M | Méthionine |
| N | Asparagine |
| P | Proline |
| Q | Glutamine |
| R | Arginine |
| S | Serine |
| T | Thréonine |
| V | Valine |
| W | Tryptophane |
| Y | Tyrosine |

On entend par séquence, tout enchaînement de motifs tels que ci-dessus définis, susceptible de constituer une séquence d'un acide nucléique ou un fragment de celui-ci d'un organisme vivant ou une séquence d'une protéine ou un fragment de celle-ci d'un organisme vivant y compris les séquences sauvages, les séquences mutantes ou encore, des séquences artificielles analogues de celles-ci obtenus par synthèse chimique ou biologique selon des méthodes connues de l'homme du métier. A titre d'exemple, et de manière non limitative, on entend par séquence contenant de tels motifs, un groupe de gènes, un gène, ou un fragment de celui-ci, un groupe de protéines, une protéine ou un fragment de celle-ci.

On entend par variante d'une séquence toute séquence différant de la séquence originale ou sauvage par au moins un motif.

Ainsi l'invention a pour objet l'identification de motifs n'ayant jamais muté simultanément parmi tous les membres d'un ensemble de séquences. L'identification de tels motifs est un enjeu majeur des nouveaux développements pharmacologiques, tant au niveau des cibles thérapeutique comme au niveau de composés thérapeutiques recherchés, notamment dans le cadre de résistances et de multirésistances développées par des organismes pathogènes nocifs tant pour l'espèce animal comme pour l'espèce végétal.

L'utilisation de fragments de séquences particulières desdits organismes pathogènes, constitués par et/ou comprenant lesdits motifs qui n'ont jamais muté simultanément en tant que composés thérapeutiques permettra, entre autres, de:
- Diminuer l'apparition de résistances aux traitements thérapeutiques;
- Stabiliser la santé du patient sur le long terme en permettant l'utilisation des médicaments disponibles sur le marché plus longtemps;
- Eviter l'apparition de maladies opportunistes ce qui diminuera le coût global du traitement;
- Diminuer la durée et le coût des investissements en recherche et développement dans l'industrie pharmaceutique.

La présente invention propose donc un nouvel outil pour optimiser le choix des traitements thérapeutiques dirigés contre des organismes pathogènes à fort taux de mutabilité ou contre des pathologies dues à l'apparition de mutations.

La méthode d'identification de motifs de l'invention consiste à comparer un sous-ensemble de variantes d'une même séquence nucléotidique ou polypeptidique d'un organisme pathogène donné, au moyen d'une séquence de référence, par exemple une séquence consensus, et à identifier lors de cette comparaison, les motifs desdites séquences qui ne mutent jamais simultanément ou les motifs qui mutent simultanément au moins une fois sur au moins une des séquences du sous-ensemble et ne mutent pas sur les autres séquences dudit sous-ensemble.

Plus précisément l'invention a pour objet une méthode d'identification d'un motif ou d'une combinaison de motifs présentant un état booléen de mutation prédéterminée dans un ensemble de séquences, comprenant les étapes suivantes;
a) l'alignement de séquences de motifs ordonnés représentées par leur code unicaractère,
b) la comparaison d'une séquence de référence à l'ensemble de séquences alignées à l'étape (a), et caractérisée en ce qu'elle comprend en outre au moins l'étape suivante :
c) l'identification des motifs n'ayant jamais muté simultanément ou des motifs ayant muté simultanément au moins une fois sur au moins une des séquences de l'ensemble et n'ayant pas muté sur les autres séquences dudit ensemble.

Selon un premier mode de réalisation de l'invention, le motif ou la combinaison de motifs à identifier est un nucléotide ou une combinaison de nucléotides et le sous-ensemble de séquences peut être extrait d'une banque de donnés d'acides nucléiques.

Selon un deuxième mode de réalisation, le motif ou la combinaison de motifs à identifier est un acide aminé ou une combinaison d'acides aminés et le sous-ensemble de séquences peut être extrait d'une banque de donnés de polypeptides et/ou de protéines.

L'alignement des séquences peut-être effectué selon toute méthode d'alignement connue de l'homme du métier.

Par exemple, lorsque le nombre de séquences du sous-ensemble que l'on utilise est inférieur à 100, on peut utiliser la méthode d'alignement Clustal W. (Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucleic Acids Research, 22:4673-4680).

Si le nombre de séquences à analyser est plus important, par exemple, supérieur à 100, l'alignement proposé par Clustal W est trop long et on peut alors avoir recours à un alignement itératif basé sur un modèle de Markov caché, ci après désigné HMM. (Sean Eddy. " Hidden Markov Models ", Curr.Opin.Struct.Biol. Vol.6, pages 361-365, 1966).

Dans ce dernier cas, il est créé, par exemple, un premier sous-ensemble de 100 séquences extraites de l'ensemble de séquences à analyser, auquel on applique la méthode de Clustal pour obtenir un premier alignement.

A partir de ce premier alignement, on crée un modèle de Markov caché (HMM) le modèle est éventuellement calibré afin de le rendre plus sensible, puis on ajoute audit premier alignement des nouvelles séquences qui seront à leur tour alignées en utilisant à nouveau HMM

Avantageusement, la séquence de référence de l'étape (b) est constituée par une séquence sauvage, ou par une séquence consensus comportant en position i le motif présent en position i dans un nombre prédéterminé des séquences de l'étape (a), par exemple dans plus de 30% desdites séquences et plus préférentiellement dans plus de 75% desdites séquences, ces valeurs pouvant être réglables selon les cas.

Avantageusement, l'étape (b) de comparaison de séquences de la méthode d'identification de l'invention consiste à:
- constituer une première matrice numérique A de dimensions NxM où N désigne le nombre de séquences et M désigne le nombre de motifs d'une des séquences dudit alignement, la valeur A_{j,i} étant égale à une première valeur A1 [par exemple "0"] lorsque le motif de position i de la séquence j est muté par rapport au motif de position i de la séquence de référence, et égale à une deuxième valeur A2 [par exemple "1"] dans les autres cas,
- constituer deux matrices d'analyse B, C des mutations où ces matrices sont :

- une matrice B de couples non mutés, c'est-à-dire de couples qui ne mutent jamais simultanément, de dimension MxM, la valeur B_{i,k} = B_{k,i} étant égale :
   - à une première valeur B1 [par exemple "0"] lorsque A_{j,i} = A_{j,k} = A1 quel que soit j allant de 0 à N,
   - à une deuxième valeur B2 [par exemple "1"] dans les autres cas;
- une matrice C de couples mutés [c'est-à-dire de couples qui mutent soit toujours, soit jamais simultanément] de dimension MxM, la valeur C_{k,i} = C_{i,k} étant égale :
   **·** à une deuxième valeur C1 [par exemple "1"] lorsque A_{j,i} = A_{j,k} quel que soit j allant de 0 à N,
   **·** à une première valeur C2 [par exemple "0"] dans les autres cas;
- à déterminer, pour un ensemble E de positions, un coefficient R_{E} dont la valeur est R₁ [par exemple "1"] lorsque toutes les valeurs B_{i,k} sont égales à la deuxième valeur B₂, quels que soient i et k appartenant à l'ensemble E desdites positions, où i ≠ k.
- à déterminer, pour un ensemble F de positions, un coefficient R_{F} dont la valeur est R₁ [par exemple "1"] lorsque toutes les valeurs C_{i,k} sont égales à la deuxième valeur C1, quels que soient i et k appartenant à l'ensemble F desdites positions, où i ≠ k.

Selon un mode de mise en oeuvre de l'invention, à l'étape (b) de la méthode, les positions des ensembles E et/ou F sont désignées par l'utilisateur.

Selon un deuxième mode de mise en oeuvre, l'étape (b) de la méthode comporte une étape de test consistant à générer la totalité des combinaisons de positions possibles et à déterminer pour chacune desdites combinaisons la valeur des coefficients R_{E} ou R_{F}, et à retenir la combinaison correspondant au plus grand ensemble de positions dont le coefficient R_{E} ou R_{F} correspond à ladite deuxième valeur.

Avantageusement, la matrice de couples mutés de l'invention permet d'identifier deux motifs ayant muté simultanément au moins une fois sur au moins une des séquences de l'ensemble et n'ayant pas muté sur les autres séquences dudit ensemble.

L'invention concerne également l'algorithme développé pour effectuer la comparaison des séquences contenant lesdits motifs et l'identification des motifs de celles-ci, soit ayant muté simultanément au moins une fois sur au moins une des séquences de l'ensemble et n'ayant pas muté sur les autres séquences dudit ensemble et consistant à :
- constituer une première matrice numérique A de dimensions NxM où N désigne le nombre de séquences et M désigne le nombre de motifs d'une des séquences dudit alignement, la valeur A_{j,i} étant égale à une première valeur A₁ [par exemple "0"] lorsque le motif de position i de la séquence j est muté par rapport au motif de position i de la séquence de référence, et égale à une deuxième valeur A₂ [par exemple "1"] dans les autres cas,
- constituer deux matrices d'analyse B, C des mutations M où cette matrice est :

- une matrice B de couples non mutés, c'est-à-dire de couples qui ne mutent jamais simultanément, de dimension MxM, la valeur B_{i,k} = B_{k,i} étant égale :
   · à une première valeur B1 [par exemple "0"] lorsque A_{j,i} = A_{j,k} = 0 quel que soit j allant de 0 à N,
   · à une deuxième valeur B2 [par exemple "1"] dans les autres cas;
- une matrice C de couples mutés [c'est-à-dire de couples qui mutent soit au moins une fois simultanément, soit jamais] de dimension MxM, la valeur C_{i,k} = C_{k,i}, étant égale :
   · à une deuxième valeur C1 [par exemple "1"] lorsque A_{j,i} = A_{j,k} quel que soit j allant de 0 à N,
   · à une première valeur C2 [par exemple "0"] dans les autres cas;
- à déterminer, pour un ensemble E de positions, un coefficient R_{E} dont la valeur est R1 [par exemple "1"] lorsque toutes les valeurs B_{i,k} sont égales à la deuxième valeur B2, quels que soient i et k appartenant à l'ensemble E desdites positions, où i ≠ k.
- à déterminer, pour un ensemble F de positions, un coefficient R_{F} dont la valeur est R1 [par exemple "1"] lorsque toutes les valeurs C_{i,k} sont égales à la deuxième valeur C2, quelles que soient i et k appartenant à l'ensemble F desdites positions, où i ≠ k.

De préférence les séquences analysées par la méthode d'identification de l'invention est constitué par un sous-ensemble de séquences extrait d'une banque des séquences nucléotidiques ou polypeptidiques d'organismes pathogènes et tout préférentiellement par des séquences nucléotidiques ou polypeptidiques d'organismes pathogènes présentant un taux élevé de mutabilité.

Selon un mode de mise en oeuvre particulier le sous-ensemble de séquences comprend toutes les séquences polypeptidiques des différentes variantes connues de la protéase du virus de l'immunodéficience humaine.

Selon une autre mise en oeuvre particulière de l'invention le sous-ensemble de séquences comprend toutes les séquences polypeptidiques des différentes variantes connues de la transcriptase inverse du virus de l'immunodéficience humaine.

Selon une autre mise en oeuvre particulière de l'invention le sous-ensemble de séquences comprend toutes les séquences polypeptidiques des différentes variantes connues de l'intégrase du virus de l'immunodéficience humaine.

L'invention concerne l'identification de motifs appartenant à tout agent pathogène dont les séquences d'acides nucléiques et/ou polypeptidiques sont susceptibles de présenter des mutations.

A titre d'exemple de telles séquences et de manière non limitative, on peut citer les séquences de virus telles que le virus de l'hépatite C qui est un virus à ARN caractérisé par la grande variabilité de son génome, avec 3% de prévalence mondiale et 600 000 personnes infectées en France, les séquences du virus ébola qui provoque des fièvres hémorragiques et qui est associé à un fort taux de mortalité, les séquences du virus de la grippe pour lequel il est nécessaire de développer de nouveaux vaccins chaque année ou les séquences de tout autre virus émergeant à fort taux de mutabilité.

Ainsi, selon une mise en oeuvre particulière de l'invention le sous-ensemble de séquences extrait comprend toutes les séquences polypeptidiques des différentes variantes de la neuraminidase du virus de la grippe.

Selon une autre mise en oeuvre particulière de l'invention le sous-ensemble de séquences extrait comprend toutes les séquences polypeptidiques des différentes variantes de l'hémagglutinine du virus de la grippe.

Aussi, parmi les séquences de bactéries susceptibles de présenter des mutations on peut également citer à titre d'exemple, la séquence C-terminal de la protéine HspA de la bactérie Helicobacter Pilori ou l'adhésine du type HA de la bactérie Escherichia Coli.

La méthode d'identification de motifs de l'invention n'est pas limitée au seul domaine des agents pathogènes. Des ensembles de séquences présentant des motifs n'ayant jamais muté simultanément, ou au contraire ayant muté simultanément au moins une fois sur au moins une des séquences de l'ensemble et n'ayant pas muté sur les autres séquences dudit ensemble, sont également présentes dans d'autres pathologies, comme par exemple, des pathologies dans le domaine de la cancérologie.

On admet, en effet qu'une grande partie des cancers est due à la présence d'éléments transposables ayant une grande homologie d'organisation avec les virus, et que le virus de l'hépatite B est la deuxième cause identifiée de mort par cancer après le tabac.

Aussi, parmi les gènes impliqués dans des cancers humains, susceptibles de présenter des motifs qui mutent et pour lesquels des ensembles de séquences ont parfois été constituées ont peut citer à titre d'exemple : le gène APC , impliqué essentiellement dans le cancer du colon (Nucleic Acids Res 1998 Jan 1;26(1):269-70, APC gene: database of germline and somatic mutations in human tumors and cell lines. Laurent-Puig P, Beroud C, Soussi T.), le gène P53 (Nucleic Acids Res 1997 Jan 1;25(1):138 p53 and APC gene mutations: software and databases. Beroud C, Soussi T.), MEN-1 (A malignant gastrointestinal stromal tumour in a patient with multiple endocrine neoplasia type 1. Papillon E, Rolachon A, Calender A, Chabre O, Barnoud R, Fournet J.), VHL (Mutations of the VHL gene in sporadic renal cell carcinoma: définition of a risk factor for VHL patients to develop an RCC. Gallou C, Joly D, Mejean A, Staroz F, Martin N, Tarlet G, Orfanelli MT, Bouvier R, Droz D, Chrétien Y, Maréchal JM, Richard S, Junien C, Beroud C.), WT1 (Clin Cancer Res 2000 Oct;6(10):3957-65. WT1 splicing alterations in Wilms' tumors. Baudry D, Hamelin M, Cabanis MO, Fournet JC, Tournade MF, Sarnacki S, Junien C, Jeanpierre C.)

L'invention a aussi pour objet l'utilisation de la méthode d'identification de motifs décrite ci-dessus pour la sélection de fragments de séquences constitués par et/ou comprenant des motifs n'ayant jamais muté simultanément pour la préparation de vaccins.

Les vaccins sont composés d'antigènes constitués par des molécules ou parties de molécules d'un organisme pathogène qui lorsqu'ils sont injectés dans l'organisme permettent de produire un plus grand nombre d'anticorps contre ledit organisme pathogène. Ces anticorps reconnaissent les molécules contre lesquelles ils sont dirigés et permettent ainsi au système immunitaire de détruire ledit organisme pathogène.

Or il s'écoule toujours un laps de temps non négligeable, parfois plusieurs années, entre le moment ou l'on définit le vaccin et le moment où il arrive sur le marché. Par exemple en ce qui concerne le virus VIH, La faible fidélité de polymérisation de la réverse-transcriptase confère au virus une grande variabilité génomique qui augmente en fonction du temps. La population virale est ainsi très hétérogène. La destruction du virus sauvage par le biais du vaccin conduit à la sélection des virus mutants contre lesquels le vaccin reste inefficace.

L'application de la méthode de l'invention à des sous-ensembles des séquences variantes des séquences protéiques de l'organisme pathogène permet de piéger ce dernier:
- Soit il mute, mais, dans ce cas, il n'est plus fonctionnel ;
- Soit, il ne mute pas, mais alors les anticorps produits à partir du vaccin permettront de le détruire.

Par exemple, en ce qui concerne le virus VIH, Les peptides faisant partie des protéines d'enveloppe du virus, identifiés parce qu'ils qui ne peuvent pas muter ensemble, probablement due à une pression génétique sous peine de perdre leur fonctionnalité, sont des candidats vaccins de choix.

En effet, la méthode d'identification de motifs peptidiques, permet de sélectionner de séquences contenant lesdits motifs, de manière contiguë ou non, afin d'élaborer un candidat vaccin. Ledit vaccin présente comme avantage, par rapport aux autres vaccins élaborées par des voies classiques, d'être décrit de façon exhaustive et de contenir de manière certaine les régions nécessaires à la stabilité dudit vaccin précisément par le choix des séquences ne pouvant pas muter ensemble simultanément, entraînant ainsi la destruction de l'organisme pathogène.

L'identification des motifs n'ayant jamais muté simultanément est plus complexe pour deux raisons principales :
- Le nombre d'acides aminés ne mutant jamais est à peu près dix fois plus grand,
- La combinaison d'acides aminés à tester n'étant pas déterminée à l'avance, toutes les combinaisons doivent être envisagées.

L'invention a également pour objet l'utilisation d'une telle méthode d'identification de motifs ou de combinaison de motifs n'ayant jamais muté simultanément pour la conception d'outils d'aide au diagnostic.

L'invention a aussi pour objet l'utilisation d'une telle méthode d'identification pour la sélection de fragments de séquence constitués par et/ou comprenant des motifs ayant toujours muté simultanément pour la préparation de tests de diagnostic.

En effet, la méthode de l'invention permet également de construire une base de connaissances qui constitue un outil d'aide à la décision, par exemple lors de la détermination par le médecin de l'administration des traitements anti-viraux à un patient donné.

Selon un autre mode de mise en oeuvre de l'invention, la méthode d'identification de motifs n'ayant jamais muté simultanément comprend une étape supplémentaire consistant à comparer des données reliant les résistances médicamenteuses connues aux mutations observées, par exemple dans les cas du VIH aux données divulguées par J. Hammond et al. dans " Mutations in Retroviral Genes Associated with Drug Resistance ". (The Human Retroviruses and AIDS Compendium. 1999)

La relation drogue-acide aminé muté, ainsi mise en évidence, est très utile pour optimiser le traitement. Par exemple, en ce qui concerne le virus VIH, la comparaison des motifs peptidiques s'effectue sur trois sous-ensembles d'une base de données protéiques, celui de la reverse transcriptase, celui de la protéase et celui de l'intégrase (http://hiv-web.lanl.gov/).

La comparaison des séquences appartenant auxdits sous-ensembles comprenant de 300 à 8000 séquences, ou de fragments desdites séquences, de chacune de ces trois protéines, permet en appliquant la méthode de l'invention, d'identifier des combinaisons d'acides aminés qui n'ont jamais muté simultanément.

Ainsi, la méthode de l'invention permet alors d'identifier les mutations induites sous la pression de sélection.

Alors la méthode de l'invention, comprenant la comparaison avec lesdites résistances médicamenteuses permet de choisir une combinaison de drogues de manière à ce que les mutations d'acides aminés susceptibles d'être provoquées par chacun des antiviraux, susceptibles de conférer la résistance aux différents médicaments impliqués dans cette combinaison (moins d'une dizaine), ne se produisent pas simultanément.

L'identification de tels motifs permet la sélection d'une combinaison médicamenteuse qui défavorise l'apparition de plus d'une mutation à la fois fermant ainsi la porte aux plurirésistances.

Le praticien pourra ensuite utiliser les informations obtenues en appliquant cette méthode par exemple aux séquences virales isolées, ou déduites du génome viral isolé, d'un patient donné pour s'assurer que la multithérapie envisagée est en effet la plus efficace possible.

L'identification d'une première mutation excluant les deux autres, une trithérapie ainsi choisie permet aux deux composés antirétroviraux restant de demeurer efficaces.

La méthode d'identification de régions peptidiques n'ayant pas muté simultanément selon l'invention apporte également une aide précieuse lors de l'apparition de résistances chez des malades déjà traités.

La méthode selon l'invention peut par exemple s'appliquer à des sous-ensembles de séquences polypeptidiques parmi lesquelles est incluse celle ou celles déduites à partir du séquençage du génome viral isolé du patient.

Ainsi, si ce génotypage met en évidence une mutation responsable de la résistance, la méthode d'identification de motifs peptidiques n'ayant pas muté permet de mettre en oeuvre une multithérapie conçue de manière à maintenir la pression de sélection sur la mutation.

La molécule ainsi sélectionnée sera accompagnée de deux ou trois autres antirétroviraux qui ciblent des domaines de la protéine ne pouvant pas muter en même temps que la zone ayant déjà muté.

Une telle méthode est utile pour la mise en oeuvre de nouvelles combinaisons antirétrovirales empêchant au maximum l'échappement thérapeutique.

Aussi, par exemple, l'identification de motifs, à l'intérieur d'un même gène, ayant muté au moins une fois simultanément sur au moins une variante et n'ayant jamais muté sur les autres variantes, permet d'identifier des régions dudit gène susceptibles de présenter une interaction physique ou fonctionnelle. En revanche, l'identification des motifs n'ayant jamais muté simultanément permet d'identifier des régions dudit gène dont la présence mutuelle est essentielle et indispensable à sa fonction.

L'invention a également pour objet l'identification, sur un ensemble de gènes ou sur un ensemble de séquences non-codantes, de motifs n'ayant jamais muté simultanément. L'identification de tels motifs permet de sélectionner des régions géniques susceptibles de présenter des interactions physiques ou fonctionnelles sur l'ensemble du génome.

Un autre objet de l'invention concerne l'utilisation d'une telle méthode d'identification de motifs ou de combinaisons de motifs pour la sélection de fragments de séquences constitués et/ou comprenant des motifs n'ayant jamais muté simultanément pour la préparation de cibles thérapeutiques.

Ainsi, la préparation, après avoir identifié des motifs n'ayant jamais muté simultanément, de fragments de séquence les contenant, permet la préparation d'une cible thérapeutique contre laquelle seront testés des composés thérapeutiques dirigés contre ledit organisme pathogène et notamment des composés thérapeutiques contre lesquels l'organisme pathogène sauvage ne pourra pas développer de mutations de résistance.

La sélection de fragments constitués et/ou comportant de motifs n'ayant jamais muté simultanément est aussi utile pour la préparation d'outils de diagnostic où il n'est pas toujours facile de détecter rapidement tel ou tel type ou sous-type d'organisme pathogène, car l'identification de motifs peptidiques selon l'invention permet la préparation des fragments de peptides comprenant les motifs les plus représentatifs d'un sous-type d'organisme pathogène. Ces fragments sont ensuite utilisés dans des tests de détection, comme des tests immunoenzymatiques, par exemple.

Cette application de la méthode de l'invention consiste à identifier un ensemble de motifs indispensable à la fonction d'une protéine d'un organisme humain, animal ou végétal ou d'un organisme pathogène. Ces motifs peuvent constituer, par exemple, un sous-ensemble d'acides aminés connus pour jouer un rôle important dans la fonction de la protéine ciblée.

Avantageusement, les motifs ainsi identifiés sont des motifs contigus de la séquence génique et représentent une séquence linéaire dudit gène.

Avantageusement, les motifs identifiés sont des motifs non contigus sur la séquence linéaire du gène. Ils peuvent alors être utiles pour compléter des études d'analyse tridimensionnelle afin de confirmer une éventuelle proximité spatiale non linéaire desdits motifs. La méthode de l'invention peut comporter alors une nouvelle étape supplémentaire (g), après l'étape (e) d'identification des motifs, consistant à comparer lesdits motifs avec les données de structures tridimensionnelles de ces protéines tels que des acides aminés impliqués dans le site catalytique et/ou dans les sites liés par des inhibiteurs non-compétitifs.

Cette dernière comparaison fournit une liste d'acides aminés impliqués dans la fonction protéique et ne mutant jamais ensemble.

La méthode d'identification de régions peptidiques selon l'invention définit les peptides les plus représentatifs d'un sous-type. Une fois identifiés, ces peptides sont utilisés dans tout test de détection connu de l'homme du métier, tels que des tests immunoenzymatiques, du type ELISA, par exemple.

La recherche de peptides représentant un sous-type d'un type particulier s'effectue comme indiqué ci-dessus. Il s'agit de trouver des antigènes peptidiques capables d'être reconnus par un sérum particulier contenant ou non les anticorps d'un sous-type particulier. La méthode selon l'invention peut s'appliquer à n'importe quelle banque de séquences, les résultats sont comparés par sous-types et la combinaison peptidique théorique la plus représentative d'un type pathogène particulier est ainsi identifiée.

Les peptides ainsi identifiés sont synthétisés et testés immunologiquement contre une collection de sérums.

L'invention présente tout son intérêt lorsqu'elle est utilisée pour identifier soit des motifs ayant muté au moins une fois ensemble, soit n'ayant jamais muté à partir d'un grand nombre de séquences comportant un grand nombre de motifs afin de sélectionner des séquences de motifs utiles pour les différentes applications envisagées ci-dessus.

Afin d'illustrer la méthode d'identification de motifs de l'invention, l'exemple ci-après montre les différentes matrices constituées lors d'une comparaison de motifs effectuée sur un sous-ensemble de huit séquences, à l'aide de la séquence de référence S V R L G H K D E V.

| POSITIONS | 0 | 1 | 2 | 3 | 4 | 5 | 6 | 7 | 8 | 9 |
|---|---|---|---|---|---|---|---|---|---|---|
| Séquence de référence (consensus) | S | V | R | L | G | H | K | D | E | V |
| | | | | | | | | | | |

| Sous-ensemble de séquences | Alignement | | | | | | | | | |
|---|---|---|---|---|---|---|---|---|---|---|
| Seq 1 | S | **R** | R | L | G | H | K | D | E | V |
| Seq 2 | S | V | R | L | G | H | K | **L** | E | V |
| Seq 3 | S | **R** | **D** | L | G | H | K | D | E | V |
| Seq 4 | S | V | R | L | G | H | **L** | D | **V** | V |
| Seq 5 | S | V | D | L | G | H | K | **T** | E | V |
| Seq 6 | S | **K** | R | L | G | H | K | D | E | V |
| Seq 7 | S | V | R | L | G | H | **G** | D | **G** | V |
| Seq 8 | S | V | R | L | G | H | K | **S** | E | V |

### 1 - MATRICE DE MUTATION A.

### Valeurs attribuées :

A1=0, si motif muté par rapport à la séquence de référence
A2=1, si autre cas (motif non muté par rapport à la séquence de référence).

| POSITION | 0 | 1 | 2 | 3 | 4 | 5 | 6 | 7 | 8 | 9 |
|---|---|---|---|---|---|---|---|---|---|---|
| Seq 1 | 1 | **0** | 1 | 1 | 1 | 1 | 1 | 1 | 1 | 1 |
| Seq 2 | 1 | 1 | 1 | 1 | 1 | 1 | 1 | **0** | 1 | 1 |
| Seq 3 | 1 | **0** | **0** | 1 | 1 | 1 | 1 | 1 | 1 | 1 |
| Seq 4 | 1 | 1 | 1 | 1 | 1 | 1 | **0** | 1 | **0** | 1 |
| Seq 5 | 1 | 1 | **0** | 1 | 1 | 1 | 1 | **0** | 1 | 1 |
| Seq 6 | 1 | **0** | 1 | 1 | 1 | 1 | 1 | 1 | 1 | 1 |
| Seq 7 | 1 | 1 | 1 | 1 | 1 | 1 | **0** | 1 | **0** | 1 |
| Seq 8 | 1 | 1 | 1 | 1 | 1 | 1 | 1 | **0** | 1 | 1 |

### 2 - MATRICE NON MUTÉE B

### Valeurs attribuées :

B1=0, si couple de motifs mutés simultanément
B2=1, si autre cas (couple de motifs jamais mutés simultanément)

| POSITION | 0 | 1 | 2 | 3 | 4 | 5 | 6 | 7 | 8 | 9 |
|---|---|---|---|---|---|---|---|---|---|---|
| POS0 | 1 | 1 | 1 | 1 | 1 | 1 | 1 | 1 | 1 | 1 |
| POS1 | 1 | **0** | 0 | 1 | 1 | 1 | 1 | 1 | 1 | 1 |
| POS2 | 1 | 0 | **0** | 1 | 1 | 1 | 1 | 0 | 1 | 1 |
| POS3 | 1 | 1 | 1 | 1 | 1 | 1 | 1 | 1 | 1 | 1 |
| POS4 | 1 | 1 | 1 | 1 | 1 | 1 | 1 | 1 | 1 | 1 |
| POS5 | 1 | 1 | 1 | 1 | 1 | 1 | 1 | 1 | 1 | 1 |
| POS6 | 1 | 1 | 1 | 1 | 1 | 1 | **0** | 1 | 0 | 1 |
| POS7 | 1 | 1 | 0 | 1 | 1 | 1 | 1 | **0** | 1 | 1 |
| POS8 | 1 | 1 | 1 | 1 | 1 | 1 | 0 | 1 | **0** | 1 |
| POS9 | 1 | 1 | 1 | 1 | 1 | 1 | 1 | 1 | 1 | 1 |

### 3 - MATRICE MUTÉE C.

### Valeurs attribuées :

C1=1, si couple de motifs mutés simultanément, ou jamais muté.
C2=0, autres cas.

| POSITION | 0 | 1 | 2 | 3 | 4 | 5 | 6 | 7 | 8 | 9 |
|---|---|---|---|---|---|---|---|---|---|---|
| POS0 | 0 | 0 | 0 | 0 | 0 | 0 | 0 | 0 | 0 | 0 |
| POS1 | 0 | 0 | 0 | 0 | 0 | 0 | 0 | 0 | 0 | 0 |
| POS2 | 0 | 0 | 0 | 0 | 0 | 0 | 0 | 0 | 0 | 0 |
| POS3 | 0 | 0 | 0 | 0 | 0 | 0 | 0 | 0 | 0 | 0 |
| POS4 | 0 | 0 | 0 | 0 | 0 | 0 | 0 | 0 | 0 | 0 |
| POS5 | 0 | 0 | 0 | 0 | 0 | 0 | 0 | 0 | 0 | 0 |
| POS6 | 0 | 0 | 0 | 0 | 0 | 0 | 0 | 0 | **1** | 0 |
| POS7 | 0 | 0 | 0 | 0 | 0 | 0 | 0 | 0 | 0 | 0 |
| POS8 | 0 | 0 | 0 | 0 | 0 | 0 | **1** | 0 | 0 | 0 |
| POS9 | 0 | 0 | 0 | 0 | 0 | 0 | 0 | 0 | 0 | 0 |

L'interrogation de la matrice mutée C permet ainsi d'identifier les motifs en positions 6 et 8 comme des motifs ayant muté au moins une fois ensemble.

## Revendications

1. Méthode mise en oeuvre par des moyens informatiques d'identification d'un motif ou d'une combinaison de motifs présentant un état booléen de mutations prédéterminées dans un ensemble de séquences comprenant les étapes suivantes :
a) l'alignement de l'ensemble de séquences de motifs ordonnés représentées par leur code unicaractère,
b) la comparaison d'une séquence de référence à l'ensemble de séquences alignées à l'étape (a), et **caractérisé en ce qu'**elle comprend en outre au moins l'étape suivante de :
c) l'identification des motifs n'ayant jamais muté simultanément ou au contraire des motifs ayant muté simultanément au moins une fois sur au moins une des séquences de l'ensemble et n'ayant pas muté sur les autres séquences dudit ensemble.

2. Méthode d'identification selon la revendication 1, **caractérisée en ce que** le motif ou la combinaison de motifs est un nucléotide ou une combinaison de nucléotides et **en ce que** le sous-ensemble de séquences est choisi parmi les séquences d'une banque de donnés d'acides nucléiques.

3. Méthode d'identification selon la revendication 1, **caractérisée en ce que** le motif ou la combinaison de motifs est un acide aminé ou une combinaison d'acides aminés et **en ce que** le sous-ensemble de séquences est choisi parmi les séquences d'une banque de donnés de polypeptides et/ou de protéines.

4. Méthode d'identification selon l'une des revendications 1 à 3, **caractérisée en ce que** la séquence de référence utilisée pour la comparaison de l'étape (b) est une séquence sauvage.

5. Méthode d'identification selon l'une des revendications 1 à 3, **caractérisée en ce que** la séquence de référence utilisée pour la comparaison de l'étape (b) est une séquence comportant en position i le motif présent en position i dans un nombre prédéterminé des séquences de l'étape (a), par exemple dans plus de 30% desdites séquences et plus préférentiellement dans plus de 75% desdites séquences.

6. Méthode d'identification selon l'une des revendications 1 à 5, **caractérisée en ce que** l'étape (b) de comparaison de séquences consiste à :
- constituer une première matrice numérique A de dimensions NxM où N désigne le nombre de séquences et M désigne le nombre de motifs d'une des séquences dudit alignement, la valeur A_{j,i} étant égale à une première valeur A1 lorsque le motif de position i de la séquence j est muté par rapport au motif de position i de la séquence de référence, et égale à une deuxième valeur A2 dans les autres cas,
- constituer deux matrices d'analyse B, C des mutations où cette matrice est :
- une matrice B de couples non mutés, c'est-à-dire de couples qui ne mutent jamais simultanément, de dimension MxM, la valeur B_{i,k} = B_{k,i} étant égale :
· à une première valeur B1 lorsque A_{j,i} = A_{j,k} = A1 quel que soit j allant de 0 à N,
· à une deuxième valeur B2 dans les autres cas;
- une matrice C de couples mutés de dimension MxM, la valeur C_{k,i} = C_{i,k} étant égale :
**·** à une deuxième valeur C1 lorsque A_{j,i} = A_{j,k} quel que soit j allant de 0 à N,
**·** à une première valeur C2 dans les autres cas;
- déterminer, pour un ensemble E de positions, un coefficient R_{E} dont la valeur est R1 lorsque toutes les valeurs B_{i,k} sont égales à la deuxième valeur B2, quels que soient i et k appartenant à l'ensemble E desdites positions, où i ≠ k.
- déterminer, pour un ensemble F de positions, un coefficient R_{F} dont la valeur est R1 lorsque toutes les valeurs C_{i,k} sont égales à la deuxième valeur C2, quels que soient i et k appartenant à l'ensemble F desdites positions, où i ≠ k.

7. Méthode d'identification selon la revendication 6, **caractérisée en ce que** à l'étape (b), les positions des ensembles E et/ou F sont désignées par l'utilisateur

8. Méthode d'identification selon l'une quelconque des revendications 6 ou 7, **caractérisée en ce que** l'étape (b) comporte une étape de test consistant à générer la totalité des combinaisons de positions possibles et à déterminer pour chacune desdites combinaisons la valeur des coefficients R_{E} ou R_{F}, et à retenir la combinaison correspondant au plus grand ensemble de positions dont le coefficient R_{E} ou R_{F} correspond à ladite deuxième valeur.

9. Méthode d'identification selon l'une quelconque des revendications 1 à 8, **caractérisée en ce que** l'ensemble de séquences analysées est constitué par des séquences de motifs d'organismes pathogènes et de préférence d'organismes pathogènes présentant un taux élevé de mutabilité.

10. Méthode d'identification selon l'une quelconque des revendications 1 à 8, **caractérisée en ce que** l'ensemble de séquences analysées est constitué par des séquences de motifs de gènes, impliqués dans des pathologies humaines, animales ou végétales et de préférence présentant un taux élevé de mutabilité.

11. Utilisation de la méthode d'identification de motifs selon l'une quelconque des revendications 1 à 10 pour la sélection de fragments de séquence constitués par et/ou comprenant des motifs n'ayant jamais muté simultanément pour la préparation de vaccins.

12. Utilisation de la méthode d'identification de motifs selon les revendications 1 à 10 pour la sélection de fragments de séquence constitués par et/ou comprenant des motifs n'ayant jamais muté simultanément pour la préparation de cibles thérapeutiques

13. Utilisation de la méthode d'identification selon les revendications 1 à 10 pour la sélection pour la sélection de fragments de séquence constitués par et/ou comprenant des motifs n'ayant jamais muté simultanément pour la préparation de tests de diagnostic.

14. Utilisation de la méthode d'identification selon les revendications 1 à 10 pour la sélection de fragments de séquence constitués par et/ou comprenant des motifs ayant toujours muté simultanément pour la préparation de tests de diagnostic.

15. Méthode d'identification de motifs, selon l'une quelconque des revendications 1 ou 3 à 10, **caractérisée en ce que** l'ensemble de séquences de l'étape (a) comprend toutes les séquences polypeptidiques des différentes variantes de la protéase du virus de l'immunodéficience humaine.

16. Méthode d'identification de motifs, selon l'une quelconque des revendications 1 ou 3 à 9, **caractérisée en ce que** l'ensemble de séquences de l'étape (a) comprend toutes les séquences polypeptidiques des différentes variantes de la transcriptase inverse du virus de l'immunodéficience humaine.

17. Méthode d'identification de motifs, selon l'une quelconque des revendications 1 ou 3 à 9, **caractérisée en ce que** l'ensemble de séquences de l'étape (a) comprend toutes les séquences polypeptidiques des différentes variantes de l'intégrase du virus de l'immunodéficience humaine.

18. Méthode d'identification de motifs, selon l'une quelconque des revendications 1 à 10, **caractérisée en ce que** l'ensemble de séquences de l'étape (a) comprend toutes les séquences de motifs des différentes variantes du gène ou de la protéine de la neuraminidase du virus de la grippe.

19. Méthode d'identification de motifs, selon l'une quelconque des revendications 1 à 10, **caractérisée en ce que** l'ensemble de séquences de l'étape (a) comprend toutes les séquences de motifs des différentes variantes du gène ou de la protéine de l'hémagglutinine du virus de la grippe.

20. Méthode d'identification de motifs, selon l'une quelconque des revendications 1 à 10, **caractérisée en ce que** l'ensemble de séquences de l'étape (a) comprend toutes les séquences de motifs des différentes variantes d'un gène et/ou d'une protéine du virus de l'hépatite C.

21. Méthode d'identification de motifs, selon l'une quelconque des revendications 1 à 10, **caractérisée en ce que** l'ensemble de séquences de motifs de l'étape (a) comprend toutes les séquences des différentes variantes des séquences du gène ou de la protéine HspA de la bactérie Helicobacter Pilori.

22. Méthode d'identification de motifs, selon l'une quelconque des revendications 1 à 10, **caractérisée en ce que** le sous-ensemble de séquences de motifs sélectionnées à l'étape (a) comprend toutes les séquences des différentes variantes du gène ou de la protéine de l'adhésine du type HA de la bactérie Escherichia Coli

23. Méthode d'identification de motifs, selon l'une quelconque des revendications 1 à 10, **caractérisée en ce qu'**elle comprend, après l'étape (c), une étape supplémentaire (d) de comparaison de motifs identifiés lors de ladite étape (c) avec les résistances médicamenteuses connues aux mutations observées.

24. Méthode d'identification de motifs, selon l'une quelconque des revendications 1 à 10, **caractérisée en ce qu'**elle comprend, après l'étape (c), une étape supplémentaire (e) de comparaison de motifs identifiés lors de ladite étape (c) avec des motifs des séquences impliqués dans un site catalytique et/ou dans des sites liés par des inhibiteurs non-compétitifs.

25. Méthode d'identification de motifs selon l'une quelconque des revendications 1 à 10, **caractérisée en ce que** la séquence identifiée est une séquence de nucléotides et **en ce qu'**elle comprend en outre une étape de synthèse de ladite séquence.

26. Méthode d'identification de motifs selon l'une quelconque des revendications 1 à 10, **caractérisée en ce que** la séquence identifiée est une séquence d'acides aminés et **en ce qu'**elle comprend en outre une étape de synthèse de ladite séquence.

27. Méthode d'identification de motifs selon l'une quelconque des revendications 15 à 21, **caractérisée en ce que** la séquence identifiée est une séquence d'acide aminés et **en ce qu'**elle comprend en outre une étape de synthèse de ladite séquence.

## Claims

1. Method implemented by computer means to identify a pattern or a combination of patterns that have a boolean state of preset mutations in a set of sequences including the following stages:
a) aligning the set of sequences of ordered patterns represented by their single character code,
b) comparing a reference sequence to the set of sequences aligned in stage (a), and **characterised in that** it additionally includes at least the following stage of:
c) identifying the patterns that have never simultaneously mutated or on the contrary the patterns that have simultaneously mutated at least once on at least one of the sequences of the set and that have not mutated on the other sequences of said set.

2. Identification method according to claim 1, **characterised in that** the pattern or combination of patterns is a nucleotide or a combination of nucleotides and that the sub-set of sequences is selected from among the sequences in a databank of nucleic acids.

3. Identification method according to claim 1, **characterised in that** the pattern or combination of patterns is an amino acid or a combination of amino acids and **in that** the sub-set of sequences is selected from among the sequences in a databank of polypeptides and/or proteins.

4. Identification method according to one of claims 1 to 3, **characterised in that** the reference sequence used for the comparison in stage (b) is a wild type sequence.

5. Identification method according to one of claims 1 to 3, **characterised** that the reference sequence used for the comparison in stage (b) is a sequence that comprises at position i the pattern present at position i in a preset number of the sequences in stage (a), for example in more than 30% of the sequences and more preferentially in more than 75% of said sequences.

6. Identification method according to one of claims 1 to 5, **characterised in that** stage (b) of comparison of sequences consists in:
- constituting a first digital matrix A of dimensions NxM where N denotes the number of sequences and M denotes the number of patterns of one of sequences of said alignment, the value Aj,i being equal to a first value A1 when the position pattern i of the sequence j is mutated relative to the position pattern i of the reference sequence, and equal to a second value A2 in the other cases,
- constituting two analysis matrices B, C of the mutations where this matrix is:
- a matrix B of non-mutated pairs, in other words pairs which never mutate simultaneously, of dimension MxM, the value B_{i,k} = B_{k,i} being equal:
to a first value B1 when Aj,i = Aj,k =
A1 whatever j may be from 0 to N,
to a second value B2 in the other cases;
- a matrix C of mutated pairs of dimension MxM, the value C_{k,i} = C_{i,k} being equal:
to a second value C1 when Aj,i = Aj,k
whatever j may be from 0 to N,
to a first value C2 in the other cases;
- determining, for a set E of positions, a coefficient R_{E} the value of which is R1 when all the values B_{i,k} are equal to the second value B2, whatever i and k may be belonging to the set E of said positions, where i = k,
- determining, for a set F of positions, a coefficient R_{F} the value of which is R1 when all the positions C_{i,k} are equal to the second value C2, whatever i and k may be belonging to the set F of said positions, where i = k.

7. Identification method according to claim 6, **characterised in that** at stage (b), the positions of the sets E and/or F are designated by the user.

8. Identification method according to any one of claims 6 or 7, **characterised in that** stage (b) comprises a test stage consisting in generating the totality of the combinations of possible positions and determining for each of said combinations the value of the coefficients R_{E} or R_{F}, and in retaining the combination that corresponds to the largest set of positions for which the coefficient R_{E} or R_{F} corresponds to said second value.

9. Identification method according to any one of claims 1 to 8, **characterised in that** the set of analysed sequences is constituted by sequences of patterns of pathogenic organisms and preferably pathogenic organisms with a high rate of mutability.

10. Identification method according to any one of claims 1 to 8, **characterised in that** the set of analysed sequences is constituted by sequences of patterns of genes, involved in human, animal or vegetable pathologies and preferably with a high rate of mutability.

11. Use of the pattern identification method according to any one of claims 1 to 10 for the selection of sequence fragments constituted by and/or including patterns that have never simultaneously mutated for the preparation of vaccines.

12. Use of the pattern identification method according to claims 1 to 10 for the selection of sequence fragments constituted by and/or including patterns that have never simultaneously mutated for the preparation of therapeutic targets.

13. Use of the identification method according to claims 1 to 10 for the selection of sequence fragments constituted by and/or including patterns that have never simultaneously mutated for the preparation of diagnostic tests.

14. Use of the identification method according to claims 1 to 10 for the selection of sequence fragments constituted by and/or including patterns that have always simultaneously mutated for the preparation of diagnostic tests.

15. Pattern identification method, according to any one of claims 1 or 3 to 10, **characterised in that** the set of sequences in stage (a) includes all the polypeptide sequences of the different variants of the protease of the human immunodeficiency virus.

16. Pattern identification method, according to any one of claims 1 or 3 to 9, **characterised in that** the set of sequences in stage (a) includes all the polypeptide sequences of the different variants of the reverse transcriptase of the human immunodeficiency virus.

17. Pattern identification method, according to any one of claims 1 or 3 to 9, **characterised in that** the set of sequences in stage (a) includes all the polypeptide sequences of the different variants of the integrase of the human immunodeficiency virus.

18. Pattern identification method, according to any one of claims 1 to 10, **characterised in that** the set of sequences in stage (a) includes all the pattern sequences of the different variants of the gene or protein of the neuraminidase of the flu virus.

19. Pattern identification method, according to any one of claims 1 to 10, **characterised in that** the set of sequences in stage (a) includes all the pattern sequences of the different variants of the gene or protein of the hemagglutinin of the flu virus.

20. Pattern identification method, according to any one of claims 1 to 10, **characterised in that** the set of sequences in stage (a) includes all the pattern sequences of the different variants of a gene and/or a protein of the hepatitis C virus.

21. Pattern identification method according to any one of claims 1 to 10, **characterised** that the set of sequences of patterns in stage (a) includes all the sequences of the different variants of the sequences of the gene or HspA protein of the bacterium Helicobacter Pilori.

22. Pattern identification method, according to any one of claims 1 to 10, **characterised in that** the sub-set of pattern sequences selected in stage (a) includes all the sequences of the different variants of the gene or protein of the type HA adhesin of the bacterium Escherichia Coli.

23. Pattern identification method, according to any one of claims 1 to 10, **characterised in that** it includes, after stage (c), an additional stage (d) of comparing patterns identified during said stage (c) with drug resistance known from observed mutations.

24. Pattern identification method, according to any one of claims 1 to 10, **characterised in that** it includes, after stage (c), an additional stage (e) of comparing patterns identified during said stage (c) with sequence patterns involved in a catalytic site and/or in sites bound by non-competitive inhibitors.

25. Pattern identification method according to any one of claims 1 to 10, **characterised in that** the identified sequence is a nucleotide sequence and **in that** it additionally includes a synthesis stage of said sequence.

26. Pattern identification method according to any one of claims 1 to 10, **characterised in that** the identified sequence is an amino acid sequence and **in that** it additionally includes a synthesis stage of said sequence.

27. Pattern identification method according to any one of claims 15 to 21, **characterised in that** the identified sequence is an amino acid sequence and **in that** it additionally includes a synthesis stage of said sequence.

## Patentansprüche

1. Verfahren, das von Informatikmitteln eingesetzt wird, zur Identifizierung eines Motivs oder einer Kombination von Motiven, die einen booleschen Zustand von vorgegebenen Mutationen in einer Gruppe von Sequenzen darstellt, die folgende Stufen umfasst:
a) die Ausrichtung der Gruppe von Sequenzen von geordneten Motiven, die durch ihren Einzeichen-Code dargestellt werden,
b) der Vergleich einer Referenzsequenz mit der Gruppe von Sequenzen, die auf Stufe (a) ausgerichtet sind und **dadurch gekennzeichnet, dass** sie außerdem mindestens folgende Stufe umfasst:
c) die Identifizierung von Motiven, die niemals gleichzeitig mutiert sind oder im Gegensatz zu den Motiven, die zumindest ein Mal in mindestens einer der Sequenzen der Gruppe gleichzeitig mutiert sind und nicht in den anderen Sequenzen der besagten Gruppe mutiert sind.

2. Verfahren zur Identifizierung gemäß Anspruch 1, **dadurch gekennzeichnet, dass** das Motiv oder die Motivkombination ein Nukleotid oder eine Kombination von Nukleotiden ist und **dadurch**, dass die Untergruppe von Sequenzen unter den Sequenzen einer Datenbank mit Nucleinsäuren ausgewählt wird.

3. Methode zur Identifizierung gemäß Anspruch 1, **dadurch gekennzeichnet, dass** das Motiv oder die Motivkombination eine Aminosäure oder eine Aminosäurekombination ist und **dadurch**, dass die Untergruppe von Sequenzen unter den Sequenzen einer Datenbank mit Polypeptiden und/oder Proteinen ausgewählt wird.

4. Verfahren zur Identifizierung gemäß einem der Ansprüche 1 bis 3, **dadurch gekennzeichnet, dass** die für den Vergleich der Stufe (b) eingesetzte Referenzsequenz eine wilde Sequenz ist.

5. Verfahren zur Identifizierung gemäß einem der Ansprüche 1 bis 3, **dadurch gekennzeichnet, dass** die für den Vergleich der Stufe (b) eingesetzte Referenzsequenz eine Sequenz ist, die an der Position i das Motiv umfasst, das an der Position i in einer vorgegebenen Anzahl von Sequenzen der Stufe (a) vorhanden ist, zum Beispiel in über 30% der besagten Sequenzen und vorzugsweise in über 75% der besagten Sequenzen.

6. Verfahren zur Identifizierung gemäß einem der Ansprüche 1 bis 5, **dadurch gekennzeichnet, dass** die Stufe (b) zum Vergleich der Sequenzen für Folgendes vorgesehen ist:
- Bilden einer ersten digitalen Matrix A mit der Abmessung NxM, wo N die Anzahl der Sequenzen bestimmt und M die Anzahl der Motive einer der Sequenzen des besagten Abgleichs bestimmt und der Wert A_{j,i} einem ersten Wert A1 entspricht, wobei das Motiv der Position i der Sequenz j im Vergleich zum Motiv der Position i der Referenzsequenz mutiert ist und einem zweiten Wert A2 in den anderen Fällen entspricht,
- Bilden von zwei Analysematrixen B, C der Mutationen, wo diese Matrix Folgendes ist:
- eine Matrix B der nicht mutierten Paare, das heißt der Paare, die niemals gleichzeitig mutieren, mit der Abmessung MxM, wobei der Wert B_{i,k} = B_{k,i} Folgendem entspricht:
einem ersten Wert B1, wenn Aj,i = Aj,k =
A1, wobei j zwischen 0 und N liegen kann,
einem zweiten Wert B2 in den anderen Fällen;
eine Matrix C der mutierten Paare mit der Abmessung MxM, wobei der Wert C_{k,i} = C_{i,k} Folgendem entspricht:
einem zweiten Wert C1, wobei A_{j,i} = A_{j,k}, wobei j zwischen 0 und N liegen kann,
einem ersten Wert C2 in den anderen Fällen;
Festlegen, für eine Gruppe E von Positionen, eines Koeffizienten R_{E}, dessen Wert R1 ist, wenn alle Werte B_{i,k} dem zweiten Wert B2 entsprechen, unabhängig von dem Wert von i und k, die zu der Gruppe E der besagten Positionen gehören, wo i = k ist.
Festlegen für eine Gruppe F von Positionen, eines Koeffizienten R_{F}, dessen Wert R1 ist, wenn alle Werte C_{i,k} dem zweiten Wert C2 entsprechen, unabhängig von dem Wert von i und k, die zu der Gruppe F der besagten Positionen gehören, wo i = k ist.

7. Verfahren zur Identifizierung gemäß Anspruch 6, **dadurch gekennzeichnet, dass** bei der Stufe (b) die Positionen der Gruppen E und/oder F von dem Benutzer bestimmt werden.

8. Verfahren zur Identifizierung gemäß einem der Ansprüche 6 oder 7, **dadurch gekennzeichnet, dass** die Stufe (b) eine Teststufe umfasst, die darin besteht, die Gesamtheit der Kombinationen der möglichen Positionen zu generieren und für jede der besagten Kombinationen den Wert der Koeffizienten R_{E} oder R_{F} zu bestimmen und die Kombination zu halten, die der größten Gruppe der Positionen entspricht, deren Koeffizient R_{E} oder R_{F} dem besagten zweiten Wert entspricht.

9. Verfahren zur Identifizierung gemäß einem der Ansprüche 1 bis 8, **dadurch gekennzeichnet, dass** die Gruppe der analysierten Sequenzen aus Sequenzen von Motiven pathogener Organismen besteht und vorzugsweise aus pathogenen Organismen mit einer hohen Veränderlichkeitsrate.

10. Verfahren zur Identifizierung gemäß einem der Ansprüche 1 bis 8, **dadurch gekennzeichnet, dass** die Gruppe der analysierten Sequenzen aus Sequenzen von Genmotiven besteht, die in menschlichen, tierlichen oder pflanzlichen Pathologien impliziert sind und vorzugsweise eine hohe Veränderlichkeitsrate aufweisen.

11. Einsatz der Methode zur Identifizierung von Motiven gemäß einem der Ansprüche 1 bis 10 für die Auswahl von Sequenzfragmenten, die aus Motiven bestehen und/oder letztere umfassen, die niemals gleichzeitig für die Aufbereitung von Impfstoffen mutiert sind.

12. Einsatz der Methode zur Identifizierung von Motiven gemäß den Ansprüchen 1 bis 10 für die Auswahl von Sequenzfragmenten, die aus Motiven bestehen und/oder letztere umfassen, die niemals gleichzeitig für die Vorbereitung therapeutischer Ziele mutiert sind.

13. Einsatz der Methode zur Identifizierung von Motiven gemäß den Ansprüchen 1 bis 10 für die Auswahl von Sequenzfragmenten, die aus Motiven bestehen und/oder letztere umfassen, die niemals gleichzeitig für die Vorbereitung von Diagnosetests mutiert sind.

14. Einsatz der Methode zur Identifizierung von Motiven gemäß den Ansprüchen 1 bis 10 für die Auswahl von Sequenzfragmenten, die aus Motiven bestehen und/oder letztere umfassen, die immer gleichzeitig für die Vorbereitung von Diagnosetests mutiert sind.

15. Methode zur Identifizierung von Motiven gemäß einem der Ansprüche 1 oder 3 bis 10, **dadurch gekennzeichnet, dass** die Gruppe der Sequenzen der Stufe (a) alle polypeptidischen Sequenzen der verschiedenen Varianten der Protease des Virus der menschlichen Immunschwäche umfasst.

16. Methode zur Identifizierung von Motiven gemäß einem der Ansprüche 1 oder 3 bis 9, **dadurch gekennzeichnet, dass** die Gruppe der Sequenzen der Stufe (a) alle polypeptidischen Sequenzen der verschiedenen Varianten der inversen Transkriptase des Virus der menschlichen Immunschwäche umfasst.

17. Methode zur Identifizierung von Motiven gemäß einem der Ansprüche 1 oder 3 bis 9, **dadurch gekennzeichnet, dass** die Gruppe der Sequenzen der Stufe (a) alle polypeptidischen Sequenzen der verschiedenen Varianten der Integrase des Virus der menschlichen Immunschwäche umfasst.

18. Methode zur Identifizierung von Motiven gemäß einem der Ansprüche 1 bis 10, **dadurch gekennzeichnet, dass** die Gruppe der Sequenzen der Stufe (a) alle Sequenzen der Motive der verschiedenen Varianten des Gens oder des Proteins der Neuraminidase des Grippevirus umfasst.

19. Methode zur Identifizierung von Motiven gemäß einem der Ansprüche 1 bis 10, **dadurch gekennzeichnet, dass** die Gruppe der Sequenzen der Stufe (a) alle Sequenzen der Motive der verschiedenen Varianten des Gens oder des Proteins der Hämaglutinine des Grippevirus umfasst.

20. Methode zur Identifizierung von Motiven gemäß einem der Ansprüche 1 bis 10, **dadurch gekennzeichnet, dass** die Gruppe der Sequenzen der Stufe (a) alle Sequenzen der Motive der verschiedenen Varianten eines Gens und/oder eines Proteins des Hepatitis-C-Virus umfasst.

21. Methode zur Identifizierung von Motiven gemäß einem der Ansprüche 1 bis 10, **dadurch gekennzeichnet, dass** die Gruppe der Sequenzen der Motive der Stufe (a) alle Sequenzen der Motive der verschiedenen Varianten der Sequenzen des Gens oder des Proteins HspA der Bakterie Heliobacter Pilori umfasst.

22. Methode zur Identifizierung von Motiven gemäß einem der Ansprüche 1 bis 10, **dadurch gekennzeichnet, dass** die Untergruppe der Motivsequenzen, die in Stufe (a) ausgewählt werden, alle Sequenzen der verschiedenen Varianten des Gens oder des Proteins des Adhesins, Typ HA, der Bakterie Escherichia Coli umfasst.

23. Methode zur Identifizierung von Motiven gemäß einem der Ansprüche 1 bis 10, **dadurch gekennzeichnet, dass** sie, nach der Stufe (c), eine zusätzliche Stufe (d) für den Vergleich der Motive umfasst, die während der besagten Stufe (c) mit den medikamentösen Widerständen, die bei den beobachteten Mutationen bekannt sind, identifiziert werden.

24. Methode zur Identifizierung von Motiven gemäß einem der Ansprüche 1 bis 10, **dadurch gekennzeichnet, dass** sie, nach der Stufe (c), eine zusätzliche Stufe (e) für den Vergleich der Motive umfasst, die während der besagten Stufe (c) mit den Motiven der Sequenzen identifiziert werden, die an einer katalytischen Stelle und/oder an den Stellen, die durch nicht wettbewerbsfähige Inhibitoren verbunden sind, impliziert werden.

25. Methode zur Identifizierung von Motiven gemäß einem der Ansprüche 1 bis 10, **dadurch gekennzeichnet, dass** die identifizierte Sequenz eine Nukleotid-Sequenz ist und **dadurch**, dass sie außerdem eine Synthesestufe der besagten Sequenz umfasst.

26. Methode zur Identifizierung von Motiven gemäß einem der Ansprüche 1 bis 10, **dadurch gekennzeichnet, dass** die identifizierte Sequenz eine Aminosäure-Sequenz ist und **dadurch**, dass sie außerdem eine Synthesestufe der besagten Sequenz umfasst.

27. Methode zur Identifizierung von Motiven gemäß einem der Ansprüche 15 bis 21, **dadurch gekennzeichnet, dass** die identifizierte Sequenz eine Aminosäure-Sequenz ist und **dadurch**, dass sie außerdem eine Synthesestufe der besagten Sequenz umfasst.
